# EUROPEAN PATENT SPECIFICATION

(11) **EP 2 358 300 B1**
(45) Date of publication and mention of the grant of the patent: **17.02.2016**
(21) Application number: 09793399.8
(22) Date of filing: 14.12.2009
(51) Int. Cl.: A61F 2/02, A61F 2/07, A61F 2/848

(54) **BARB FOR ANCHORING AN IMPLANTABLE MEDICAL DEVICE WITHIN A BODY VESSEL**
WIDERHAKEN ZUR VERANKERUNG EINES MEDIZINISCHEN IMPLANTATS IN EINEM KÖRPERGEFÄSS
BARBE D'ANCRAGE D'UN DISPOSITIF MÉDICAL IMPLANTABLE DANS UN VAISSEAU DU CORPS

(30) Priority: 17.12.2008 US 138355 P
(43) Date of publication of application: 24.08.2011
(73) Proprietor: Cook Medical Technologies LLC, Bloomington, IN 47404 (US)
(72) Inventor: LEEWOOD, Alan, R., Lafayette IN 47905 (US); YANG, Shuo, West Lafayette IN 47906 (US); DIERKING, William, K., Louisville KY 40222 (US); SWIFT, Richard, A., South Bend IN 46635 (US); ROEDER, Blayne, A., Lafayette IN 47909 (US)
(74) Representative: Powell, Stephen David
(86) International application number: PCT/US2009/067848
(87) International publication number: WO 2010/077808

(56) References cited:
- WO-A1-00/35352
- WO-A1-01/76509
- WO-A1-2005/072652
- WO-A1-2008/088716
- US-A- 5 344 427
- US-A- 5 891 160

## Description

### Technical Field

The present disclosure is related generally to implantable medical devices and more particularly to the fixation of implantable medical devices within body vessels.

### Background

Aneurysms occur in blood vessels at sites where, due to age, disease or genetic predisposition of the patient, the strength or resilience of the vessel wall is insufficient to prevent ballooning or stretching of the wall as blood passes through. If the aneurysm is left untreated, the blood vessel wall may expand and rupture, often resulting in death.

To prevent rupturing of an aneurysm, a stent graft may be introduced into a blood vessel percutaneously and then deployed to span the aneurysmal sac. A stent graft includes a graft fabric secured to a cylindrical scaffolding or framework of one or more stents. The stents provide rigidity and structure to hold the graft open in a tubular configuration as well as the outward radial force needed to create a seal between the graft and a healthy portion of the vessel wall. Blood flowing through the vessel can be channeled through the hollow interior of the stent graft to reduce, if not eliminate, the stress on the vessel wall at the location of the aneurysmal sac. Thus, a stent graft may reduce the risk of rupture of the blood vessel wall at the aneurysmal site and allow blood to flow through the vessel without interruption.

Aneurysms occurring in the aorta, the largest artery in the human body, may occur in the chest (thoracic aortic aneurysm) or in the abdomen (abdominal aortic aneurysm). The high velocity of blood within the aorta makes it essential to securely anchor the stent graft to the aortic wall. Migration of the stent graft downstream is prevented, in part, by a frictional interference fit between the vessel wall and an oversized sealing stent at the proximal end of the graft. The integrity of the interference fit may be compromised, however, by changes in the size and morphology of the vessel. Consequently, stent grafts may include anchoring barbs that penetrate the aortic wall to provide additional resistance to migration that is not dependent on the interference fit.

Anchoring barbs experience repetitive loading from hemodynamic forces that are a by-product of the cardiac cycle. Exposure to cyclic loading may lead to premature fracture of the barbs due to fatigue failure.

WO 00/35352 A1 discloses a barb for anchoring an implantable medical device within a body vessel, the barb comprising a thin-walled body portion for engagement with a structural element of an implantable medical device, the body portion comprising a tip portion for anchoring into tissue and a base portion between the tip portion and the body portion, where, in a deployed configuration of the barb, the base portion curves away from the longitudinal axis at a radius of curvature and the tip portion is substantially straight.

### Disclosure of the Invention

An improved barb for anchoring an implantable medical device within a body vessel is described herein. The improved barb is designed to have a higher fatigue life than existing barbs.

According to a first aspect of the invention, there is provided a barb as defined in claim 1.
The first curvature may have a non constant or variable radius of curvature. The second curvature may have a non constant or variable radius of curvature.

The geometry of the improved barbs distributes the strain over a larger region of the penetrating element compared to the existing barbs. Accordingly, the peak maximum principal strain is decreased. Increasing the length of the base portion of the penetrating element results in a decrease in the maximum principal strain - and thus an improvement in the fatigue life of the barb. The base portion preferably extends over from about 25% to about 50% of the total length of the penetrating element. Advantageously the base portion extends over from about 33% to about 45% of the total length of the penetrating element. A reduction in the curvature of the base portion is also beneficial for reducing the maximum principal strain. Preferably, the peak maximum principal strain experienced by the barb is about 0.5% or less when a longitudinally-directed load of 1.5 N is applied to a midpoint of the penetrating element.

### Brief Description of the Drawings

Preferred embodiments of the invention will now be described with reference to the drawings in which:
Figure 1 shows an example useful in explaining the present invention of a barb for anchoring an implantable medical device within a body vessel;
Figure 2 shows an embodiment in accordance with the present invention of a barb for anchoring an implantable medical device within a body vessel; Figure 3A shows contours of maximum principal strain in a prior art barb, where the maximum principal strain is determined by finite element analysis (FEA) of the barb under load;
Figure 3B shows contours of maximum principal strain for the barb of Figure 1 when under load, where the maximum principal strain is determined by finite element analysis (FEA);
Figure 3C shows contours of maximum principal strain for the barb of Figure 2 when under load, as determined by FEA;
Figures 3D and 3E show contours of maximum principal strain for modified versions of the barb of Figure 1 when under load, as determined by FEA;
Figure 4 shows a transverse cross-sectional view of a body portion of the barb of Figure 1 or 2 engaged with a structural element of an implantable medical device;
Figure 5 shows a transverse cross-sectional view of another body portion of a barb engaged with a structural element of an implantable medical device;
Figure 6 shows a transverse cross-sectional view of another body portion of a barb engaged with a structural element of an implantable medical device;
Figures 7A-7C show an exemplary process for forming the barb of Figure 1 ; and
Figures 8A-8C show an exemplary process for forming the barb of Figure 2.

### Description of the Preferred Embodiments

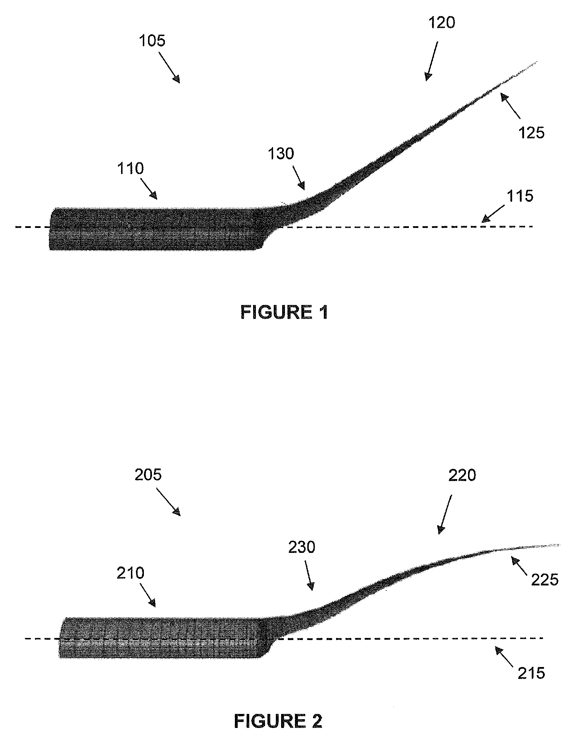

Figures 1 and 2 show barbs 105, 205 for anchoring an implantable medical device within a body vessel. Each barb 105, 205 is shown in a deployed configuration suitable for anchoring into tissue. When secured to an implantable medical device, as discussed further below, such barbs 105, 205 help prevent or minimize migration of the implantable medical device within the body vessel.

Referring to Figure 1 , the example of the barb 105 useful in explaining the present invention includes a thin- walled body portion 110 for engagement with a structural element of an implantable medical device. A penetrating element 120 extends from the body portion 110 of the barb 105. The penetrating element 120 includes a tip portion 125 for anchoring into tissue and a base portion 130 between the tip portion 125 and the body portion 110. The body portion 110 defines a longitudinal axis 115. In the deployed configuration, the base portion 130 of the penetrating element 120 curves away from the longitudinal axis 115 at a nonconstant radius of curvature, and the tip portion 125 is substantially straight. That is, the tip portion 125 has a substantially constant slope with respect to the longitudinal axis. In an undeployed configuration of the barb 105, the penetrating element 120 preferably extends in a longitudinal direction from the body portion 110 so as to have a low- profile configuration.

The base portion 130 may extend over as much as about half of the length of the penetrating element 120, so that up to about 50% of the length of the penetrating element 120 curves away from the longitudinal axis 115 at a non- constant radius of curvature. The tip portion 125 extends over the remaining portion of the length of the penetrating element 120; thus, at least about 50% of the length of the penetrating element 120 may be substantially straight, or have a substantially constant slope with respect to the longitudinal axis. Preferably, the base portion 130 extends over at least about one-quarter (25%) of the length of the penetrating element 120, and the tip portion 125 extends over no more than about three-quarters (75%) of the length of the tip portion 125. For example, the base portion 130 may extend over at least about one-third (33%) of the length of the penetrating element 120, and the tip portion 125 may extend over no more than about two-thirds (67%) of the length of the penetrating element 120. In another example, the base portion 130 may extend over at least about 40% of the length of the penetrating element 120, and the tip portion 125 may extend over no more than about 60% of the length of the penetrating element 120. Other proportions of the base portion 130 and tip portion 125 along the length of the penetrating element 120 are also possible, and are determined by the forming process, as discussed in detail below.

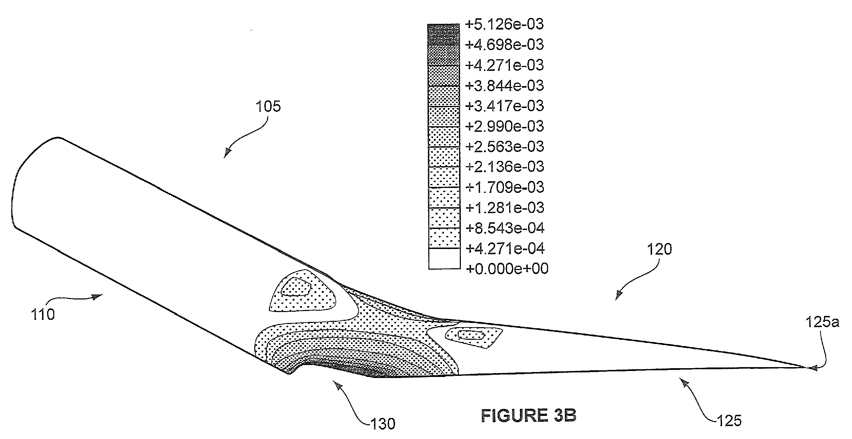

As shown in Figure 3B, the penetrating element 120 preferably has a decreasing width along its length from the base portion 130 to the tip portion 125. The width of the penetrating element 120 may decrease to zero or nearly zero at the end 125a of the tip portion 125 so as to define a sharp tip 125a effective for penetrating tissue. Accordingly, the tip 125a may have a radius of curvature of zero or nearly zero. It is also envisioned that the penetrating element 120 may have a spade-like configuration with a width that initially increases and then decreases along the length from the base portion 130 to the tip portion 125.

Figure 2 shows an embodiment of the barb 205 in accordance with the present invention for anchoring an implantable medical device within a body vessel. As in the above example, the barb 205 includes a thin-walled body portion 210 for engagement with a structural element of an implantable medical device, and a penetrating element 220 extends from the body portion 210. The penetrating element 220 includes a tip portion 225 for anchoring into tissue and a base portion 230 between the tip portion 225 and the body portion 210. The body portion 210 defines a longitudinal axis 215. In the deployed configuration, the base portion 230 of the penetrating element 220 curves away from the longitudinal axis 215 at a first curvature, and the tip portion 225 curves toward the longitudinal axis at a second curvature opposite in sign from the first curvature. A first radius of curvature corresponding to the first curvature may be nonconstant. A second radius of curvature corresponding to the second curvature may also be nonconstant. In an undeployed configuration of the barb 205, the penetrating element 220 preferably extends in a longitudinal direction from the body portion 210 so as to have a low-profile configuration.

The base portion 230 may extend over as much as about half of the length of the penetrating element 220, so that up to about 50% of the length of the penetrating element 220 curves away from the longitudinal axis 215. The tip portion 225, which curves toward the longitudinal axis, extends over the remaining portion of the length of the penetrating element 220. Preferably, the base portion 230 extends over at least about one-quarter (25%) of the length of the penetrating element 220, and the tip portion 225 extends over no more than about three-quarters (75%) of the length of the tip portion 225. For example, the base portion 230 may extend over at least about one-third (33%) of the length of the penetrating element 220, and the tip portion 225 may extend over no more than about two-thirds (67%) of the length of the penetrating element 220. In another embodiment, the base portion 230 may extend over at least about 40% of the length of the penetrating element 220, and the tip portion 225 may extend over no more than about 60% of the length of the penetrating element 220. Other proportions of the base portion 230 and tip portion 225 along the length of the penetrating element 220 are also possible, and are determined by the forming process, as discussed in detail below.

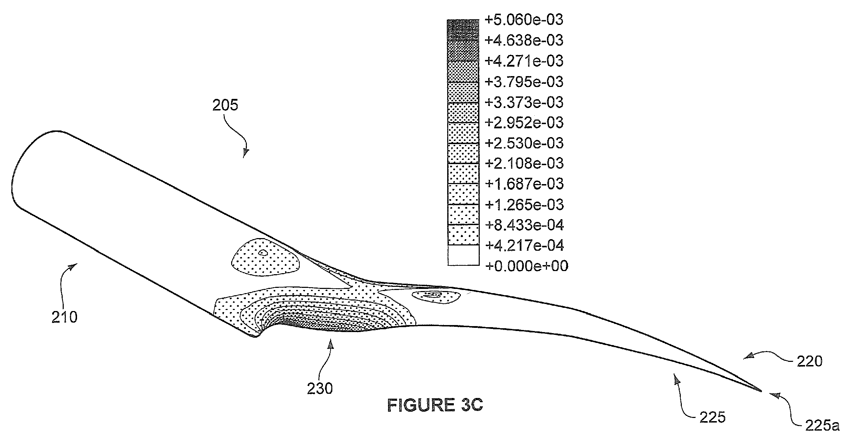

As in the previous embodiment, the penetrating element 220 preferably has a decreasing width along its length from the base portion 230 to the tip portion 225. Referring to Figure 3C, the width of the penetrating element 220 may decrease to zero or nearly zero at the end of the tip portion 225 so as to define a sharp point 225a effective for penetrating tissue. Accordingly, the sharp point 225a may have a radius of curvature of zero or nearly zero. Alternatively, the penetrating element 220 may have the spade-like configuration described above.

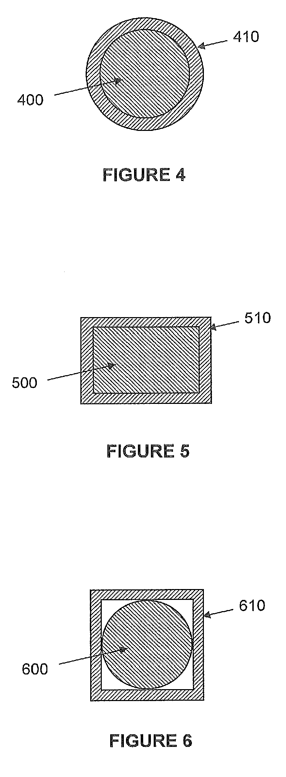

The thin-walled body portion 110, 210 of the barbs 105, 205 may take the form of a sleeve sized to at least partially surround a structural element of an implantable medical device. The sleeve may completely surround the structural element, as shown in Figures 4-6, which show a transverse cross-sectional view of an exemplary thin-walled body portion engaged with a structural element. Depending on the shape of and means of attachment to the structural element, the sleeve may have a tubular shape (e.g., a "cannula") or a non-tubular shape. For example, as shown in Figure 4, the sleeve 410 may be formed from a thin-walled tube or from a thin-walled curved sheet that is sized to fit over a round wire 400. The sleeve may have a substantially round cross section. Alternatively, as shown in Figures 5 and 6, the sleeve 510, 610 may be formed by bending a thin-walled sheet or by crimping a thin-walled tube into a shape that is sized to fit over a flat wire 500 or a round wire 600. The flat wire 500 may be a strut of a laser-cut stent. The flat wire 100 may have a substantially rectangular cross section. The sleeve may have a substantially rectangular cross section. It is also possible for the sleeve to only partially surround the structural element of the implantable medical device.

The thin-walled body portion may be secured to the structural element of the implantable medical device by one or more of an adhesive bond, a thermal bond, a spot weld, a laser weld, a frictional fit, a crimp or another joining mechanism.

The barb may be formed in whole or in part from a shape memory alloy that can "remember" and recover a previous shape. In the case of nickel-titanium shape memory alloys (e.g., Nitinol), the source of the shape recovery is a phase transformation between a lower temperature phase (martensite) and a higher temperature phase (austenite), which may be driven by a change in temperature (shape memory effect) or by the removal of an applied stress (superelastic effect). Strain introduced into the alloy in the martensitic phase to achieve a shape change may be substantially recovered upon completion of a reverse phase transformation to austenite, allowing the alloy to return to the previous shape. Austenite is characteristically the stronger phase, and martensite is the more readily deformable phase which may accommodate recoverable strains of up to about 8-10%.

Slightly nickel-rich Nitinol alloys including, for example, about 51 at.% Ni and about 49 at.% Ti are known to be useful for intraluminal medical devices that are superelastic at body temperature. In particular, alloys including 50.6 - 50.8 at.% Ni and 49.2 - 49.4 at.% Ti are considered to be medical grade Nitinol alloys and may be used for the barbs. The nickel-titanium alloy may also include one or more additional alloying elements that may substitute for one or both of the nickel and titanium in the alloy.

It may be particularly advantageous for the barb to be able to "remember" and recover the deployed configuration, where the penetrating element extends away from the longitudinal axis to facilitate tissue penetration, after delivery to an intraluminal treatment site. Accordingly, the barb may undergo a heat-setting treatment during processing that programs a memory of the desired deployed configuration. Thus, the barb (and the medical device to which the barb is affixed) may be delivered into a body vessel in a low-profile delivery configuration and then superelastically deployed to the remembered configuration once in place at the treatment site. A tubular restraining sheath overlying the medical device and the barb(s) may be retracted to deploy the device and barb superelastically.

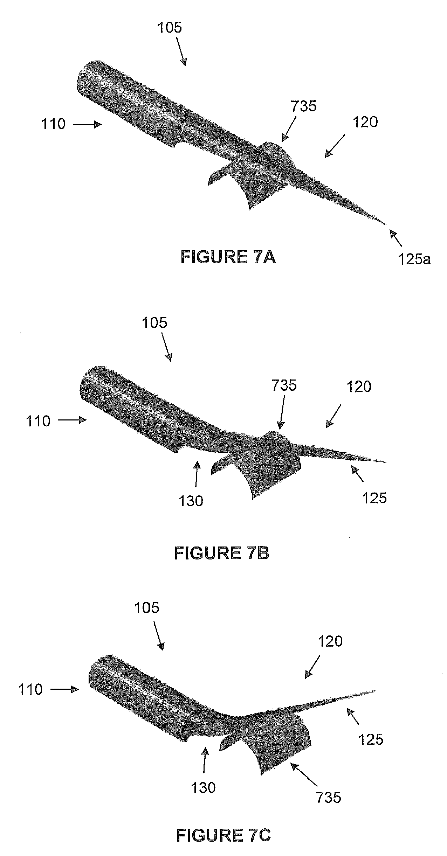

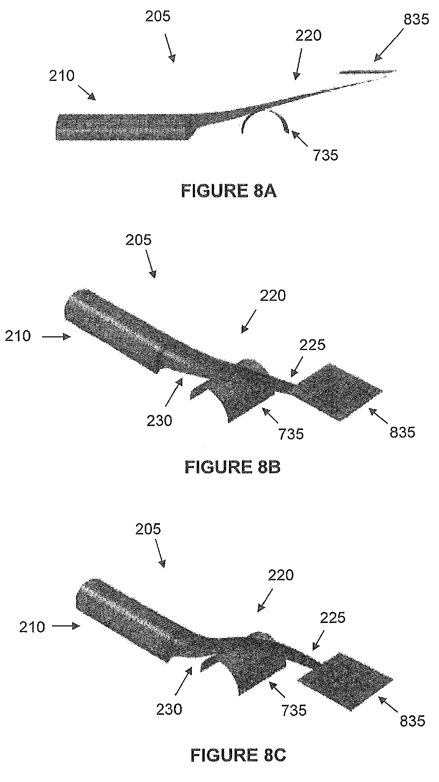

Set forth herein are proposed methods of forming the barbs into the desired deployed configurations prior to heat-setting. Figures 7A through 7C show a method of forming the example of Figure 1, and Figures 8A through 8C show a method of forming the embodiment of Figure 2.

Referring to Figures 7A through 7C, a forming die 735 may be placed adjacent to a lower surface of the penetrating element 120 and then moved vertically or radially outward in order to obtain the desired deployed configuration of the barb 105. The forming die 735 may be a mandrel made of a tool steel or another hard material, where a longitudinal axis of the mandrel is oriented perpendicular to that of the barb 105. According to this example, the base portion 130 of the penetrating element 120 is curved away from the longitudinal axis of the barb 105 at a nonconstant radius of curvature by the motion of the forming die 735, while the tip portion 125 of the penetrating element 120 remains straight. The positioning of the forming die 735 along the length of the penetrating element determines the extent of the base portion 130 relative to the tip portion 125. By positioning the forming die 735 closer to the tip 125a, a longer base portion 130 may be formed, and by positioning the forming die 735 farther from the tip 125a, a longer tip portion 125 may be formed. In Figures 7A-7C, the forming die is positioned at a "2/3 span location" along the penetrating element 120 as measured from the tip 125a. By moving the forming die 735 against the penetrating element 120 at this location, the barb, an example of which is shown in Figure 1 , may be obtained.

Referring to Figures 8A through 8C, a first forming die 735 and a second forming die 835 may be employed to form the embodiment of the barb 205. The first forming die 735 may be placed adjacent to a lower surface of the penetrating element 220 and then moved upward or radially outward in order to curve the base portion 230 of the penetrating element 220 away from the longitudinal axis of the barb 205. As described previously, the first forming die 735 may be a mandrel having a longitudinal axis oriented perpendicular to that of the barb 105. The second forming die may have the shape of a plate, as shown in Figures 8A-8C, or it may be a mandrel similar to the first forming die. The second forming die 835 contacts an upper surface of the tip portion 225 of the penetrating 220 as the first forming die 735 is moved upward or outward against the lower surface of the base portion 230. The positioning of the first forming die 735 along the length of the penetrating element determines the extent of the base portion 230 relative to the tip portion 225. As in Figures 7A-7C, the forming die 735 is positioned at a "2/3 span location" along the penetrating element 220 as measured from the tip. By moving the first forming die 735 against the penetrating element 220 with the forming die 835 positioned as described, the second embodiment of the barb 205, an example of which is shown in Figure 2, may be obtained.

Once formed into the desired configuration, the heat-setting treatment mentioned above may be carried out to program the barb with a memory of the configuration. The barb may be constrained by one or more fixtures, mandrels or other suitable constraining members to hold the desired configuration during the heat-setting treatment, which is typically carried out at a temperature in the range of from about 350°C to about 550°C.

The heat setting treatment may be carried out for a time duration of from about 15 minutes to about 60 minutes.

Due to the repetitive loading experienced by barbs in vivo, it is useful to understand how design variations may affect their fatigue performance. The inventors have employed finite element analysis (FEA) using Abaqus/Standard 6.8-1 software to predict the behavior of different Nitinol barb configurations under stress. A 1.5 N load was applied in a longitudinal direction to a midpoint of the penetration element of each barb as modeled by FEA. A material model for Nitinol which is widely accepted in the medical device community was used in the FEA simulations.

From the FEA simulations, it is possible to determine the maximum principal strain experienced by each barb during loading. The strain contours reveal local regions of the barbs that are most highly strained during loading. The peak maximum principal strain is believed to be an indicator of fatigue life; in general, the lower the magnitude of the peak strain, the more fatigue-resistant or durable the barb. The inventors compared the maximum principal strain values for the improved barb configurations described herein to those achieved for a previous barb design disclosed in U.S. Patent Application Publication No. 2005/0240259.

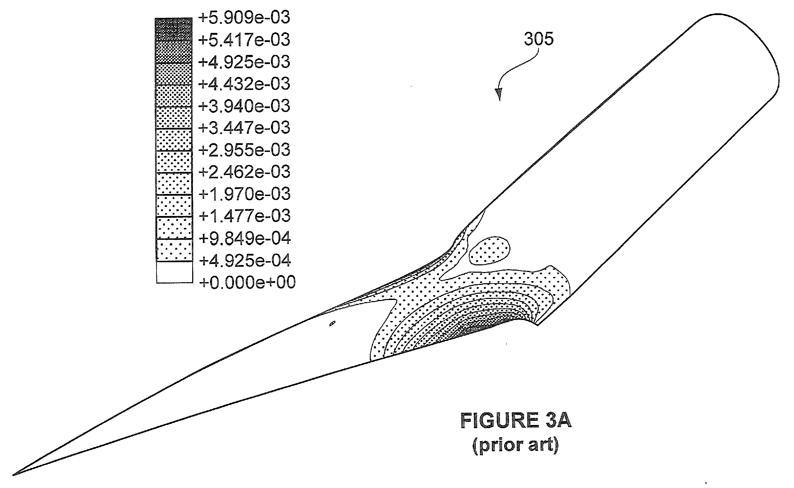

Figures 3A-3C show contours of maximum principal strain in the loaded barbs. Figure 3A depicts the previous barb design, and Figures 3B and 3C show barb configurations according to the example and embodiment discussed above. In particular, Figure 3B shows a barb having a penetrating element 120 with a base portion 130 that curves away from the longitudinal axis 115 of the barb at a nonconstant radius of curvature and a tip portion 125 that is substantially straight. Figure 3C shows a barb having a penetrating element with a base portion 230 that curves away from the longitudinal axis 215 of the barb at a first curvature, and a tip portion 225 that curves toward the longitudinal axis at a second curvature opposite in sign from the first curvature. Each base portion 130, 230 extends over about 33% of the length of the penetrating element, and each tip portion extends over about 67% of the length of the penetrating element. Such proportions may be created with a (first) forming die located at a 2/3 span location along the penetrating element 120, 220 with respect to the tip 125a, 225a. As the contours of maximum principal strain in the Figures indicate, when loaded as described above, the strain is concentrated in the base portion 130, 230 of each barb 105, 205.

Referring to Figure 3A, the peak maximum principal strain experienced by the previous barb configuration (the "baseline case") when under the load of 1.5 N is 0.591%, or about 0.6%.

Referring to Figure 3B, the peak maximum principal strain experienced by the barb of the example is 0.513%, a decrease of over 13% compared to that obtained for the baseline case. Referring to Figure 3C, the peak maximum principal strain experienced by the embodiment of the improved barb is even further reduced. In this case, the barb experiences a strain of 0.506%, which is a decrease of over 14% compared to the baseline case.

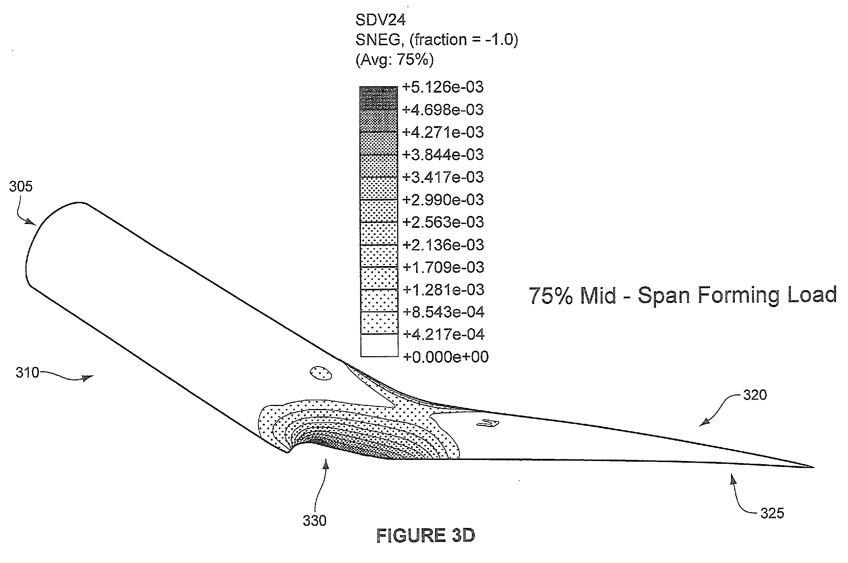

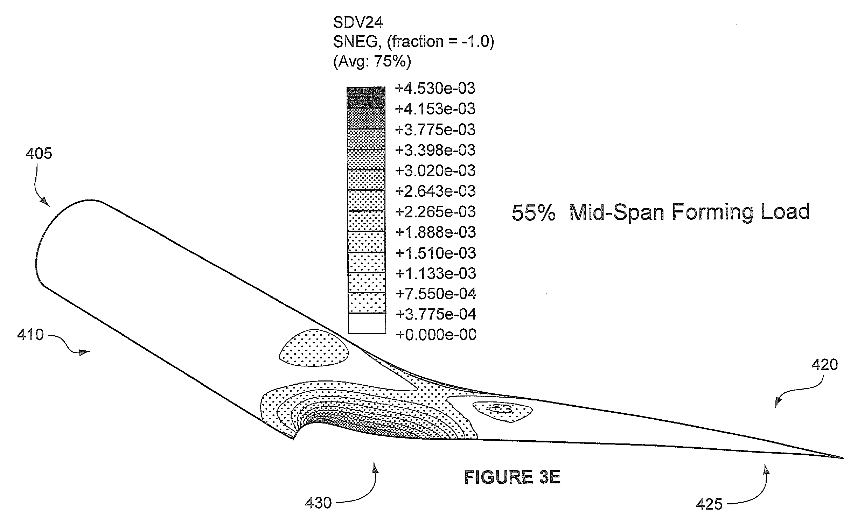

Figures 3D and 3E show the maximum principal strain experienced by the barb of the example when the barb is created using a alternative forming die positions. In Figure 3D, for example, the barb 305 is created using a forming die positioned at a 75% span location along the penetrating element 320 as measured from the tip 325a, such that the base portion 330 extends over about 25% of the length of the penetrating element 320 and the tip portion 325 extends over about 75% of the length. In Figure 3E, the barb 405 is created by a forming die positioned at a 55% span location along the penetrating element 420 as measured from the tip 425a, such that the base portion 430 extends over about 45% of the length of the penetrating element 420 and the tip portion 425 extends over about 55% of the length. The barb 305 of Figure 3D experiences a peak maximum principal strain of 0.513% under the 1.5 N load, and the barb 405 of Figure 3E experiences a peak maximum principal strain of 0.453% under the same conditions. The latter strain value is over 23% lower than the peak strain experienced by the baseline barb design.

The FEA data suggest that the geometry of the improved barbs distributes the strain over a larger region of the penetrating element compared to the baseline case. Accordingly, the peak maximum principal strain is decreased. It is believed that by further increasing the length of the base portion of the penetrating element, where the base portion has the curvature of either the first or the second embodiment of the barb, a decrease in the maximum principal strain -- and thus an improvement in the fatigue life of the barb - may be achieved. As described above, the base portion preferably extends over from about 25% to about 50% of the total length of the penetrating element. It may be particularly advantageous for the base portion to extend over from about 33% to about 45% of the total length of the penetrating element. A reduction in the curvature of the base portion may also be beneficial for reducing the maximum principal strain. Preferably, based on the above data, the peak maximum principal strain experienced by the barb is about 0.5% or less when a longitudinally-directed load of 1.5 N is applied to a midpoint of the penetrating element.

An improved barb for anchoring an implantable medical device to a body vessel has been described. The improved barb is designed to have a higher fatigue life than existing barbs. Although the present invention has been described in considerable detail with reference to certain embodiments thereof, other embodiments are possible without departing from the present invention. The scope of the appended claims should not be limited, therefore, to the description of the preferred embodiments contained herein. All embodiments that come within the meaning of the claims, either literally or by equivalence, are intended to be embraced therein. Furthermore, the advantages described above are not necessarily the only advantages of the invention, and it is not necessarily expected that all of the described advantages will be achieved with every embodiment of the invention.

## Claims

1. A barb (205) for anchoring an implantable medical device within a body vessel, the barb comprising:
a thin-walled body portion (210) for engagement with a structural element of an implantable medical device, the body portion having a longitudinal axis;
a penetrating element (220) extending in a longitudinal direction from the body portion in an undeployed configuration of the barb and comprising a tip portion (225) for anchoring into tissue and a base portion (230) between the tip portion and the body portion, where, in a deployed configuration of the barb, the base portion curves away from the longitudinal axis at a first radius of curvature and the tip portion curves towards the longitudinal axis at a second curvature opposite in sign from the first, and wherein the base portion extends over about 25% to 50% of a total length of the penetrating element.

2. A barb as claimed in claim 1 wherein the first curvature has a variable first radius of curvature over the base portion (230).

3. A barb as claimed in claim 1 or 2 wherein the second curvature has a variable second radius of curvature over the tip portion (225).

4. A barb as claimed in any preceding claim wherein the penetrating element (220) comprises a maximum strain of about 0.5% or less when subjected to a load of 1.5 N, the load being applied in a longitudinal direction to a midpoint of the penetrating element.

5. The barb of any preceding claim wherein the base portion (230) extends over at least about 33% of the total length of the penetrating element.

6. The barb of claim 5 wherein the base portion (230) extends over at least about 40% of the total length of the penetrating element.

7. The barb of any of claims 1 to 4 wherein the base portion (230) extends over about 45% of the total length of the penetrating element.

8. The barb of any preceding claim wherein the penetrating element (220) has a decreasing width along a length thereof from the base portion to the tip portion.

9. The barb of any preceding claim wherein the penetrating element (220) has a width that initially increases and then decreases along the length from the base portion to the tip portion.

10. The barb of any preceding claim wherein the thin-walled body portion (210) comprises a sleeve (410; 510; 610) sized to at least partially surround the structural element of the implantable medical device.

11. The barb of claim 10 wherein the sleeve (410; 510; 610) has a substantially round or rectangular cross section.

12. The barb of any preceding claim wherein the penetrating element (220) is formed of a superelastic nickel-titanium alloy.

## Patentansprüche

1. Widerhaken (205) zur Verankerung eines medizinischen Implantats in einem Körpergefäß, wobei der Widerhaken Folgendes umfasst:
einen dünnwandigen Körperabschnitt (210) für Eingriff mit einem Strukturelement eines medizinischen Implantats, wobei der Körperabschnitt eine Längsachse aufweist;
ein penetrierendes Element (220), das sich in einer Längsrichtung von dem Körperabschnitt in einer nicht eingesetzten Konfiguration des Widerhakens erstreckt und einen Spitzenabschnitt (225) zur Verankerung in Gewebe und einen Basisabschnitt (230) zwischen dem Spitzenabschnitt und dem Körperabschnitt aufweist, worin sich der Basisabschnitt in einer eingesetzten Konfiguration des Widerhakens von der Längsachse in einem ersten Krümmungsradius weg biegt und sich der Spitzenabschnitt zur Längsachse in einem zweiten Krümmungsradius mit umgekehrtem Vorzeichen vom ersten biegt, und worin sich der Basisabschnitt über ungefähr 25% bis 50% einer Gesamtlänge des penetrierenden Elements erstreckt.

2. Widerhaken nach Anspruch 1, worin die erste Krümmung einen variablen ersten Krümmungsradius über dem Basisabschnitt (230) aufweist.

3. Widerhaken nach Anspruch 1 oder 2, worin die zweite Krümmung einen variablen zweiten Krümmungsradius über dem Spitzenabschnitt (225) aufweist.

4. Widerhaken nach einem der vorhergehenden Ansprüche, worin das penetrierende Element (220) eine maximale Dehnung von ungefähr 0,5% oder weniger umfasst, wenn es einer Last von 1,5 N ausgesetzt ist, wobei die Last in einer Längsrichtung auf einen Mittelpunkt des penetrierenden Elements aufgebracht wird.

5. Widerhaken nach einem der vorhergehenden Ansprüche, worin sich der Basisabschnitt (230) über mindestens ungefähr 33% der Gesamtlänge des penetrierenden Elements erstreckt.

6. Widerhaken nach Anspruch 5, worin sich der Basisabschnitt (230) über mindestens ungefähr 40% der Gesamtlänge des penetrierenden Elements erstreckt.

7. Widerhaken nach einem der Ansprüche 1 bis 4, worin sich der Basisabschnitt (230) über mindestens ungefähr 45% der Gesamtlänge des penetrierenden Elements erstreckt.

8. Widerhaken nach einem der vorhergehenden Ansprüche, worin das penetrierende Element (220) eine abnehmende Breite entlang einer Länge davon von dem Basisabschnitt zu dem Spitzenabschnitt aufweist.

9. Widerhaken nach einem der vorhergehenden Ansprüche, worin das penetrierende Element (220) eine Breite aufweist, die anfänglich zunimmt und dann entlang der Länge von dem Basisabschnitt zu dem Spitzenabschnitt abnimmt.

10. Widerhaken nach einem der vorhergehenden Ansprüche, worin der dünnwandige Körperabschnitt (210) eine Hülle (410; 510; 610) umfasst, die eine solche Größe aufweist, dass sie das Strukturelement des medizinischen Implantats mindestens teilweise umgeben kann.

11. Widerhaken nach Anspruch 10, worin die Hülle (410; 510; 610) einen im Wesentlichen runden oder rechteckigen Querschnitt aufweist.

12. Widerhaken nach einem der vorhergehenden Ansprüche, worin das penetrierende Element (220) aus einer superelastischen Nickel-Titan-Legierung geformt ist.

## Revendications

1. Barbe (205) pour ancrer un dispositif médical implantable à l'intérieur d'un vaisseau corporel, la barbe comprenant:
une partie de corps à paroi mince (210) à engager avec un élément structurel d'un dispositif médical implantable, la partie de corps présentant un axe longitudinal; et
un élément de pénétration (220) qui s'étend dans une direction longitudinale à partir de la partie de corps dans une configuration non déployée de la barbe, et comprenant une partie de pointe (225) à ancrer dans un tissu et une partie de base (230) entre la partie de pointe et la partie de corps, dans laquelle, dans une configuration déployée de la barbe, la partie de base s'incurve à l'écart de l'axe longitudinal à un premier rayon de courbure, et la partie de pointe s'incurve en direction de l'axe longitudinal à un deuxième rayon courbure dont le signe est opposé au premier, et dans laquelle la partie de base s'étend sur environ 25 % à 50 % d'une longueur totale de l'élément de pénétration.

2. Barbe selon la revendication 1, dans laquelle la première courbure présente un premier rayon de courbure variable sur la partie de base (230).

3. Barbe selon la revendication 1 ou 2, dans laquelle la deuxième courbure présente un deuxième rayon de courbure variable sur la partie de pointe (225).

4. Barbe selon l'une quelconque des revendications précédentes, dans laquelle l'élément de pénétration (220) présente une déformation maximum d'environ 0,5 % ou moins lorsqu'il est soumis à une charge de 1,5 N, la charge étant appliquée dans une direction longitudinale en un point médian de l'élément de pénétration.

5. Barbe selon l'une quelconque des revendications précédentes, dans laquelle la partie de base (230) s'étend sur au moins environ 33 % de la longueur totale de l'élément de pénétration.

6. Barbe selon la revendication 5, dans laquelle la partie de base (230) s'étend sur au moins environ 40 % de la longueur totale de l'élément de pénétration.

7. Barbe selon l'une quelconque des revendications 1 à 4, dans laquelle la partie de base (230) s'étend sur environ 45 % de la longueur totale de l'élément de pénétration.

8. Barbe selon l'une quelconque des revendications précédentes, dans laquelle l'élément de pénétration (220) présente une largeur décroissante le long d'une longueur de celui-ci à partir de la partie de base jusqu'à la partie de pointe.

9. Barbe selon l'une quelconque des revendications précédentes, dans laquelle l'élément de pénétration (220) présente une largeur qui augmente initialement et qui diminue ensuite le long de la longueur à partir de la partie de base jusqu'à la partie de pointe.

10. Barbe selon l'une quelconque des revendications précédentes, dans laquelle la partie de corps à paroi mince (210) comprend un manchon (410; 510; 610) dimensionné de manière à entourer au moins partiellement l'élément structurel du dispositif médical implantable.

11. Barbe selon la revendication 10, dans laquelle le manchon (410; 510; 610) présente une section transversale sensiblement ronde ou rectangulaire.

12. Barbe selon l'une quelconque des revendications précédentes, dans laquelle l'élément de pénétration (220) est constitué d'un alliage super-élastique de nickel-titane.
